# FASCICULE DE BREVET EUROPEEN

(11) **EP 0 585 804 B1**
(45) Date de publication et mention de la délivrance du brevet: **14.10.1998**
(21) Numéro de dépôt: 93113613.9
(22) Date de dépôt: 26.08.1993
(51) Int. Cl.: A61K 7/04

(54) **Composition coricide contenant du NaOH**
NaOH enthaltende Hühneraugenmittel
Corn remover containing NaOH

(30) Priorité: 03.09.1992 CH 2772/92
(43) Date de publication de la demande: 09.03.1994
(73) Titulaire: SALTRATES INTERNATIONAL S.A., 1208 Genève (CH)
(72) Inventeur: Viret, Jean-Louis, CH-1817 Fontanivent (CH)
(74) Mandataire: Hranitzky, Wilhelm Max

(56) Documents cités:
- EP-A- 0 437 225
- WO-A-92/13533
- FR-A- 1 238 980

## Description

La présente invention concerne une composition coricide contenant, en tant que substance active kératolytique, de l'hydroxyde de sodium en solution aqueuse, en présence d'une amine agissant comme substance tampon à l'égard de l'hydroxyde de sodium.

La demande de brevet européen EP-A-0.437.225 décrit des préparations topiques contenant du 1α,25-dihydroxycholecalciférol, en tant qu'ingrédient actif pharmaceutique et mentionne l'utilisation du tris(hydroxyméthyl)aminométhane en tant que tampon pour ajuster le pH de la préparation à une valeur comprise en 6,5 et 7,5 à partir d'une valeur initiale légèrement acide qui lui est conférée par le véhicule pharmaceutique.

Le brevet français No. 1.238.980 décrit une composition destinée à amollir les callosités constituée d'une solution aqueuse d'alcanolamine contenant de la soude ou de la potasse et pouvant éventuellement contenir d'autres composés tels que des agents désinfectants, anesthésiques ou rafraîchissants. Ce brevet mentionne à titre d'exemple d'alcanolamine pouvant être utilisée, la monoéthanolamine, la monobutanolamine, la monopropanolamine, la diéthanolamine et la triéthanolamine.

Un tel produit coricide se présentant sous forme d'un gel et dans lequel la substance tampon est constituée par de la triéthanolamine, est bien connu et utilisé, avec succès, depuis de nombreuses années.

Dans ce produit, la triéthanolamine qui est une base faible, bien tolérée par la peau et sans effet kératolytique, atténue l'agressivité de l'hydroxyde de sodium vis-à-vis de la peau, grâce au fait qu'elle exerce un effet tampon sur cette base forte, tout en maintenant une action kératolytique suffisante à l'égard des parties durcies, à éliminer, de la peau.

L'avancement des connaissances dans le domaine de la chimie, ainsi qu'en ce qui concerne les effets des substances chimiques sur l'organisme de l'homme et des mammifères supérieurs, a toutefois récemment mis en évidence le fait que la triéthanolamine est susceptible d'être partiellement nitrolysée, dans certaines conditions, et notamment en présence de nitrites, en formant des nitrosamines, qui pourraient avoir un effet cancérigène sur l'homme et les mammifères.

Bien que, jusqu'à présent, on n'a rapporté aucune observation permettant de suspecter que le produit coricide connu susmentionné pourrait effective ment être cancérigène, il est apparu justifié de mettre au point une nouvelle formulation d'un tel produit ne contenant plus de triéthanolamine. Cela afin d'éliminer tout risque d'effet cancérigène pouvant résulter d'une éventuelle formation de nitrosamines par nitrolysation de cette substance.

L'invention a donc pour objectif de fournir une composition coricide du genre indiqué ci-dessus, dans laquelle la triéthanolamine est remplacée par une autre amine ayant un effet tampon à l'égard de l'hydroxyde de sodium, mais n'étant pas susceptible d'être nitrolysée.

A cet effet, la composition coricide selon l'invention présente les caractéristiques spécifiées dans la revendication 1.

Avantageusement, la composition coricide est sous forme d'un gel.

De préférence, la teneur en hydroxyde de sodium de la composition coricide selon l'invention est de 0,5 à 8 % en poids et sa teneur en tris (hydroxyméthyl)-aminométhane est de 0,5 à 15 % en poids. Plus particulièrement, les gammes de teneurs les plus avantageuses en hydroxyde de sodium et en tris (hydroxyméthyl)aminométhane sont, respectivement, de 1,5 à 3 %, en poids, et de 3 à 10 %, en poids.

Avantageusement, la composition peut contenir au moins une substance gélifiante, ayant pour fonction de lui conférer une viscosité appropriée et, éventuellement, au moins un agent rhéologique destiné à lui donner des propriétés thyxotropiques, en vue d'assurer que, grâce à une élévation suffisante du seuil d'écoulement du produit, la quantité utilisée pour une application sur un cor reste en place pendant toute la durée requise pour l'obtention de l'effet kératolytique souhaité.

En outre, il s'est révélé avantageux d'incorporer à la composition au moins un agent ayant pour effet de renforcer et de stabiliser l'action de ladite substance gélifiante et dudit agent rhéologique.

Comme substance gélifiante, on peut notamment utiliser une gomme cellulosique, telle qu'une hydroxyéthylcellulose (en particulier le produit commercialisé sous la marque NATROSOL 250 HR et fabriqué par la société HERCULES INCORPORATED, Wilmington, USA).

Comme agent rhéologique, on peut utiliser, par exemple, du silicate de magnésium et d'aluminium (en particulier le produit commercialisé sous la marque VEEGUM HS et fabriqué par la société VANDERBILT COMPANY, INC., Norwalk, USA).

En tant qu'agent ayant pour effet de renforcer et stabiliser l'action de la substance gélifiante et celle de l'agent rhéologique, on peut utiliser, par exemple, un copolymère d'acide acrylique ou d'acide méthacrylique et d'un ester d'acide méthacrylique et de polyéthylène glycol éther d'alcool stéarique, tel que le produit connu sous la dénomination technique "ACRYLATES/ STEARETH-20 METHACRYLATE COPOLYMER" et commercialisé sous la marque PRIMAL ICS-1 ainsi que sous la marque ACRYSOL ICS-1, ce produit étant fabriqué par la société ROHM and HAAS COMPANY, Philidelphia, USA.

D'autre part, la composition peut avantageusement comprendre au moins un agent tensio-actif, de même qu'au moins un agent bactéricide, au moins une substance ayant un effet anesthésique local, au moins une substance aromatisante ainsi que toute autre substance additionnelle utile, compatible avec les autres constituants de cette composition.

Comme agent bactéricide, on peut notamment utiliser le chlorure de cétylpyridinium, qui exerce également un effet tensio-actif.

Comme substance à effet anesthésique local, on peut, par exemple, utiliser le produit connu sous la dénomination technique "LAURETH-9" (selon le "CTFA International Cosmetic Ingredient Dictionary") et commercialisé sous la marque POLIDOCANOL, ce produit étant fabriqué par la société ZIMMERLI S.A., Aarbourg, Suisse. Le "LAURETH-9" est un éther polyglycolique d'alcool laurique, c.à.d. un hydroxy-polyéthoxy-dodécane contenant, en moyenne, 9 motifs oxyéthylène par molécule. Il est à remarquer que ce produit exerce, outre son action anesthésiante, un effet tensio-actif.

L'effet tensio-actif des additifs susmentionnés permet d'améliorer l'adhérence de la composition sur la peau, ce qui favorise notablement l'action de la substance kératolytique et, par conséquent, a pour effet de raccourcir le temps de traitement.

Comme substance aromatisante, on peut, par exemple, utiliser du menthol.

On va maintenant donner, à titre non limitatif, un exemple de la composition coricide selon l'invention et décrire un mode de préparation de cette composition.

### EXEMPLE:

La nature et les teneurs des ingrédients d'une composition coricide selon l'invention, qui se présente sous forme d'un gel à la température ambiante, sont, par exemple, les suivantes, les teneurs étant exprimées en pourcentage pondéral:

| | |
|---|---|
| Eau | 81,77 |
| Tris (hydroxyméthyl) aminométhane | 5,00 |
| Substance à effet anesthésique local "LAURETH-9" (produit commercialisé sous la dénomination "POLIDOCANOL") | 5,00 |
| Hydroxyéthylcellulose (produit commercialisé sous la dénomination "NATROSOL 250 HR") | 3,70 |
| Hydroxyde de sodium | 2,00 |
| Silicate de magnésium et d'aluminium (produit commercialisé sous la dénomination "VEEGUM HS") | 1,50 |
| Copolymère d'acide acrylique et d'ester d'acide méthacrylique et de polyéthylène glycol éther d'alcool stéarique (produit commercialisé sous la dénomination "PRIMAL ICS-1") | 1,00 |
| Chlorure de cétylpyridinium | 0,02 |
| Menthol | 0,01 |

Ce produit se présente sous forme d'un gel homogène, lisse et brillant, de couleur très légèrement grise-brunâtre, ayant une très faible odeur de menthe.

Ce produit a un pH de 12,38 et il présente les valeurs de mesure de rhéologie suivantes, selon l'essai normalisé DIN 125 :
- 36.10³ à 38.10³ mPa.s: (pour un taux de cisaillement de 10,133)
- 26.10³ à 27.10³ mPa.s: (pour un taux de cisaillement de 20,000)
ainsi que des valeurs de mesure de thixotropie de 900 à 1500 Pa/s.

Pour la préparation de ce produit, on procède de la manière suivante:

Dans un réacteur muni d'un rotor tournant à grande vitesse, servant de dispositif homogénéisateur, on prépare un mélange homogène de 75.77 parties en poids d'eau et 1,50 parties en poids de silicate de magnésium et d'aluminium (VEEGUM HS).

On obtient un mélange parfaitement lisse et homogène, exempt de bulles d'air, en effectuant le traitement d'homogénéisation pendant 10 à 15 minutes pour une vitesse de rotation de 18.000 tours/minute, sans refroidir la masse de mélange au cours de ce traitement. A la masse ainsi obtenue, on ajoute ensuite, lentement, par saupoudrage, 3,70 parties en poids de poudre d'hydroxyéthylcellulose (NATROSOL 250 HR), tout en brassant la masse au moyen d'un brasseur à ancre, tournant à une vitesse de 100 à 200 tours/minute.

Après incorporation complète de l'hydroxyéthylcellulose dans la masse, on ajoute encore, successivement, en poursuivant le brassage, 1 partie en poids de copolymère d'acrylate/méthacrylate (PRIMAL ICS-1) en poudre, puis un mélange de 5 parties en poids de "LAURETH-9" et 0,01 partie en poids de menthol pur et, finalement, une solution de chlorure de cétylpyridinium (0,02 partie en poids) dans 2 parties en poids d'eau, et on continue le brassage jusqu'à ce que le mélange soit parfaitement homogène.

On laisse alors reposer la masse pendant environ 15 à 20 heures afin de laisser complètement gonfler l'hydroxyéthylcellulose.

Après quoi, on ajoute à la masse 2 parties en poids d'hydroxyde de sodium, en solution dans 4 parties en poids d'eau, tout en brassant lentement la masse au moyen d'un brasseur-racleur tournant à environ 50 tours par minute. La durée de cette opération est d'environ 10 à 20 minutes, jusqu'à obtention d'un mélange homogène.

Finalement, on ajoute à ce dernier mélange, tout en continuant le brassage au moyen du brasseur-racleur, 5 parties en poids de tris (hydroxyméthyl)-aminométhane pur (de qualité pharmaceutique) jusqu'à ce que le produit soit parfaitement homogène.

On obtient ainsi le produit dont la composition a été indiquée cidessus.

Ce produit présente, lorsqu'on l'acidifie au moyen d'acide chlorhydrique 1-n, deux points d'inflexion, dont le premier, obtenu pour une quantité de 0,42 ml d'HCl 1-n par gramme de produit, correspond à une valeur de pH égale à 9,41 et le second, obtenu pour une quantité de 0,97 ml d'HCl 1-n par gramme de produit, correspond à une valeur de pH égale à 4,93.

La stabilité chimique du produit est excellente comme l'ont montré des mesures des valeurs desdits points d'inflexion effectuées après maintien d'échantillons de ce produit pendant un an à des températures égales à 20, 37 et 47°C, respectivement, dans des emballages fermés en matières différentes (verre ou matière plastique). Ces mesures n'ont permis de constater que des variations très faibles de ces valeurs.

Des essais de vieillissement portant sur l'aspect et l'odeur du gel, le pH du produit, sa viscosité et ses propriétés thixotropiques ont également permis de constater une excellente stabilité de ces propriétés et caractéristiques, surtout dans le cas où le produit est conservé dans un récipient en verre.

Le produit qui vient d'être décrit a une excellente activité coricide combinée avec une parfaite innocuité et il peut être utilisé de la même manière que le produit coricide de l'art antérieur mentionné au début de la description.

Plus précisément, la composition coricide selon l'invention est principalement destinée à l'utilisation dans le traitement local d'appoint des cors et des durillons.

Pour le traitement des cors, on peut avantageusement procéder en appliquant, avec précision, sur le cor, de préférence en utilisant une canule d'application, après un bain des pieds et séchage parfait du pied, la quantité juste suffisante de composition, sous forme d'un gel, pour recouvrir toute la surface du cor traité, en prenant soin de ne pas faire déborder le gel sur la peau saine environnant le cor, et en laissant agir la composition pendant 5 à 10 minutes, après quoi on rince soigneusement à l'eau tiède et on sèche à nouveau le pied.

## Revendications

1. Composition coricide contenant, en tant que substance active kératolytique, de l'hydroxyde de sodium, en solution aqueuse, en présence d'une amine agissant comme substance tampon à l'égard de l'hydroxyde de sodium, caractérisée par le fait que cette substance tampon est constituée par le tris (hydroxyméthyl)-aminométhane.

2. Composition coricide selon la revendication 1, caractérisée par le fait qu'elle est sous forme d'un gel.

3. Composition coricide selon la revendication 1 ou la revendication 2, caractérisée par le fait que sa teneur en hydroxyde de sodium est de 0,5 à 8 % en poids et sa teneur en tris (hydroxyméthyl)-aminométhane est de 0,5 à 15 % en poids.

4. Composition coricide selon l'une des revendications précédentes, caractérisée par le fait qu'elle contient au moins une substance gélifiante.

5. Composition selon la revendication 4, caractérisée par le fait que ladite substance gélifiante est une hydroxyméthylcellulose.

6. Composition coricide selon l'une des revendications précédentes, caractérisée par le fait qu'elle contient au moins un agent rhéologique.

7. Composition selon la revendication 6, caractérisée par le fait que ledit agent rhéologique est un silicate de magnésium et d'aluminium.

8. Composition selon la revendication 4 et la revendication 6, caractérisée par le fait qu'elle contient au moins un agent ayant pour effet de renforcer et stabiliser l'action de la substance gélifiante et celle de l'agent rhéologique.

9. Composition selon la revendication 8, caractérisée par le fait que ledit agent renforçant et stabilisant l'action de la substance gélifiante et de l'agent rhéologique est un copolymère d'acide acrylique ou d'acide méthacrylique et d'ester d'acide méthacrylique et de polyéthylène glycol éther d'alcool stéarique.

10. Composition selon l'une des revendications précédentes, caractérisée par le fait qu'elle contient au moins un agent tensio-actif, et/ou au moins un agent bactéricide, et/ou au moins une substance exerçant un effet anesthésique local.

## Claims

1. Corn-curing composition containing, as a keratolytic active substance, sodium hydroxide in aqueous solution, in the presence of an amine acting as a buffering substance with respect to sodium hydroxide, characterized by the fact that this buffering substance is formed of tris (hydroxymethyl) aminomethane.

2. Corn-curing composition according to claim 1, characterized by the fact that it is in the form of a gel.

3. Corn-curing composition according to claim 1 or to claim 2, characterized by the fact that its sodium hydroxide content is of 0.5 to 8 percent in weight and its content in tris (hydroxymethyl) aminomethane is of 0.5 to 15 percent in weight.

4. Corn-curing composition according to one of the preceding claims, characterized by the fact that it contains at least one gelling substance.

5. Corn-curing composition according to claim 4, characterized by the fact that said gelling substance is a hydroxy-methyl cellulose.

6. Corn-curing composition according to one of the preceding claims, characterized by the fact that it contains at least one rheological agent.

7. Composition according to claim 6, characterized by the fact that said rheological agent is a silicate of magnesium and aluminium.

8. Composition according to claim 4 and to claim 6, characterized by the fact that it contains at least one agent producing an increase and stabilization of the action of the gelling substance and of that of the rheological agent.

9. Composition according to claim 8, characterized by the fact that said agent increasing and stabilizing the action of the gelling substance and of the rheological agent is a copolymer of acrylic acid or methacrylic acid and of methacrylic acid ester and of polyehtylene glycol ether of stearic alcohol.

10. Composition according to one of the preceding claims, characterized by the fact that it contains at least one surface active agent and/or at least one bacteriolytic agent and/or at least one substance having a local anaesthetic effect.

## Patentansprüche

1. Komposition zur Hühneraugenbekämpfung, die als aktive keratolytische Substanz Natriumhydroxid in wässriger Lösung enthält, in Gegenwart eines Amins, das gegenüber dem Natriumhydroxid als Puffersubstanz wirkt, dadurch gekennzeichnet, dass diese Puffersubstanz durch Tris (Hydroxymethyl)-Aminomethan gebildet wird.

2. Komposition zur Hühneraugenbekämpfung gemäss Patentanspruch 1, dadurch gekennzeichnet, dass sie in Form eines Gels ist.

3. Komposition zur Hühneraugenbekämpfung gemäss Patentanspruch 1 oder Patentanspruch 2, dadurch gekennzeichnet, dass ihr Gehalt an Natriumhydroxid 0,5 bis 8 Gewichtsprozent und ihr Gehalt an Tris (Hydroxymethyl)-Aminomethan 0,5 bis 15 Gewichtsprozent beträgt.

4. Komposition zur Hühneraugenbekämpfung gemäss einem der vorhergehenden Patentansprüche, dadurch gekennzeichnet, dass sie mindestens eine gelbindende Substanz enthält.

5. Komposition zur Hühneraugenbekämpfung gemäss Patentanspruch 4, dadurch gekennzeichnet, dass die genannte gelbindende Substanz eine Hydroxymethyl-Zellulose ist.

6. Komposition zur Hühneraugenbekämpfung gemäss einem der vorhergehenden Patentansprüche, dadurch gekennzeichnet, dass sie mindestens ein Viskositätsmittel enthält.

7. Komposition gemäss Patentanspruch 6, dadurch gekennzeichnet, dass das genannte Viskositätsmittel ein Magnesium- und Aluminium-Silikat ist.

8. Komposition gemäss Patentanspruch 4 und Patentanspruch 6, dadurch gekennzeichnet, dass sie mindestens ein Mittel enthält, das eine Verstärkung und Stabilisierung der Wirkung der gelbindenden Substanz und der Wirkung des Viskositätsmittels bewirkt.

9. Komposition gemäss Patentanspruch 8, dadurch gekennzeichnet, dass das genannte, die Wirkung der gelbindenden Substanz und des Viskositätsmittels verstärkende und stabilisierende Mittel ein Copolymer von Acrylsäure oder Methacrylsäure und Methacrylsäureester und Stearylalkohol-Polyäthylenglykoläther ist.

10. Komposition nach einem der vorhergehenden Patentansprüche, dadurch gekennzeichnet, dass sie mindestens ein die Oberflächenspannung reduzierendes Mittel und/oder mindestens ein Bakterizid und/oder mindestens eine Substanz, die lokal-anästhesierend wirkt, enthält.
